# EUROPEAN PATENT SPECIFICATION

(11) **EP 2 065 035 B1**
(45) Date of publication and mention of the grant of the patent: **21.07.2010**
(21) Application number: 07380332.2
(22) Date of filing: 28.11.2007
(51) Int. Cl.: A61K 9/20, A61K 31/415

(54) **Pharmaceutical formulations containing irbesartan**
Pharmazeutische Formulierungen mit Irbesartan
Formules pharmaceutiques contenant de l'irbesartan

(43) Date of publication of application: 03.06.2009
(73) Proprietor: Laboratorios Lesvi, S.L., 08970 Sant Joan Despi (Barcelona) (ES)
(72) Inventor: González Pérez, Marino, 08004 Barcelona (ES); Ubeda Pérez, Carmen, 08348 Cabrils - Barcelona (ES); Díez Martín, Ignacio, 08980 Sant Feliu de Llobregat - Barcelona (ES)
(74) Representative: ABG Patentes, S.L.

(56) References cited:
- EP-A- 0 747 050
- WO-A-2005/118166
- DE-A1-102005 031 577

## Description

### FIELD OF THE INVENTION

The present invention relates to new pharmaceutical compositions and formulations for the oral administration of Irbesartan, one of its pharmaceutically acceptable salts or its polymorphs, optionally combined with a diuretic and to a process for the manufacture of said composition.

### BACKGROUND OF THE INVENTION

Irbesartan is an oral selective Angiotensin II antagonist pharmaceutically active compound useful for the treatment of hypertension and heart failure. The chemical name of Irbesartan is 2-n-butyl-4-spirocyclopentane-1-[((2'-tetrazol-5-yl)biphenyl-4-yl)methyl]-2-imidazolin-5-one with CAS Registry number 138402-11-6 and structural formula **I.**

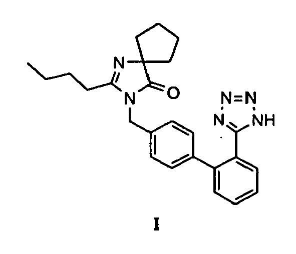

The synthesis of Irbesartan is disclosed in EP0454511B1, EP0708103B1 and EP1853591 among other patent and patent applications.

Irbesartan is marketed as Aprovel® in 75, 150 and 300 mg alone and as Coaprovel® in combination with hydrochlorothiazide (HCTZ, with structural formula **II)** in 150/12.5 mg and 300/25 mg strengths:

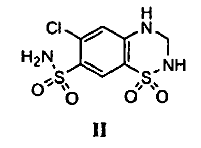

In EP0708103 there are described two polymorphic forms of Irbesartan, the polymorphic Forms A and B.

Different formulations of Irbesartan are disclosed in the art.

EP0747050A describes pharmaceutical formulations containing 20-70% irbesartan and a poloxamer as a surfactant to enhance dissolution. Granules of Irbesartan, diluent, binder, disintegrant and surfactant where prepared via wet granulation process using water.

EP1089994A describes pharmaceutical compositions containing Irbesartan in a specific crystalline habit prepared according to EP0747050.

EP1750862A describes pharmaceutical compositions containing more than 70% Irbesartan and a surfactant. Irbesartan is granulated with a solution of the binder and further granulated with more binder and the surfactant.

EP 1806130A describes surfactant free Irbesartan·HCl formulations containing a disintegrant and a sugar alcohol as a diluent, Irbesartan is granulated with many ingredients, including mannitol, using alcohol 96%.

EP 1670461 describes Irbesartan formulations containing a poloxamer (surfactant). The Irbesartan is granulated with the surfactant using alcohol 96%.

EP1275391 describes pharmaceutical compositions of Irbesartan and a diuretic. Irbesartan and the diuretic are granulated with part of the diluent, part of the disintegrant and the binder using a wet granulation process with water.

WO2007099555 describes irbesartan formulations with lactose and free of surfactant.

As stated in EP0747050B1, Irbesartan shows certain physical properties that challenge the preparation of a suitable formulation containing the required amount of drug (75-300 mg) in a small easily to swallow tablet. Irbesartan is a fluffy material with low density, and then is difficult to handle it to prepare reproducible tablets. To obtain a good bioavailability, it is desired to obtain irbesartan tablets with total disgregation in less than 30 min.

For all these reasons, there is still a need in the art for developing pharmaceutical compositions with small size which incorporate Irbesartan and methods suitable for industrial scale for preparing said compositions with an improved physical stability which allows desirable disgregation time and bioavailability properties. Furthermore, these new formulations need to allow preparing these tablets easily at an industrial scale.

### BRIEF DESCRIPTION OF THE INVENTION

The authors of the present invention have surprisingly found that a composition comprising Irbesartan as active ingredient, and maltose as diluent, allows obtaining homogeneous oral pharmaceutical formulations, such as tablets, with also a good flowability. In particular, the best results are obtained when Irbesartan is firstly granulated with a binder and a disintegrant and then mixed with maltose and other excipients. The use of maltose improves the results when compared to those obtained with lactose as diluent, since formulations with lactose result in bad fluidity and nonhomogeneous formulations and also they could not be tabletted.

Therefore, in a first aspect the present invention relates to a pharmaceutical composition comprising Irbesartan **characterized in that** it includes maltose.

In a second aspect, the present invention relates to an oral pharmaceutical formulation which comprises the pharmaceutical composition as defined above.

In a particular embodiment, the oral pharmaceutical formulation comprises:
a. granules comprising Irbesartan, a binder and a disintegrant; and
b. a mixture of maltose, a disintegrant, a glidant, a lubricant and, optionally, an additional diluent:

In another particular embodiment, the pharmaceutical formulation further comprises a diuretic inside the granules and/or out the granules.

A third aspect of the present invention is a process for preparing an oral pharmaceutical formulation as defined above, more preferably a tablet, which comprises mixing and granulating the Irbesartan, the disintegrant and, optionally, the binder with water or a water solution of the binder to obtain the granules.

In a particular embodiment, this process further comprises: a) mixing the granules previously obtained with maltose, the glidant, the disintegrant and, optionally, the additional diluent and the diuretic; b) mixing the mixture obtained in step a) with the lubricant; c) compressing the resulting mixture obtained in step b) to prepare a tablet; and d) optionally, coating the tablet.

In another particular embodiment, the process further comprises mixing and granulating with the diuretic. In this case, the process further comprises: a) mixing the granules previously obtained with maltose, the glidant, the disintegrant, and, optionally, the additional diluent; b) mixing the mixture obtained in step a) with the lubricant; c) compressing the resulting mixture obtained in step b) to prepare a tablet; and d) optionally, coating the tablet.

### DETAILED DESCRIPTION OF THE INVENTION

In the present invention the maltose is considered as defined in the US National Formulary 25.

The present invention relates to a pharmaceutical composition comprising Irbesartan as active ingredient and maltose as a diluent. This composition allows obtaining oral pharmaceutical compositions, preferably tablets.

In a particular embodiment of the invention, the oral pharmaceutical formulation is characterized by comprising:
a) granules comprising Irbesartan, a binder and a disintegrant;
b) a mixture of maltose, a disintegrant, a glidant, a lubricant and, optionally, an additional diluent.

In a particular embodiment, the pharmaceutical formulation further comprises a diuretic into the granules and/or out of the granules.

More preferably, the granules comprise 67 to 97.5% by weight of Irbesartan; 2 to 12% by weight of a binder; 0.5 to 6% by weight of a disintegrant; and 0 to 15% by weight of a diuretic, said percentages being with respect to the total weight of the granules.

In another preferred embodiment, the oral pharmaceutical formulation is characterized by the following total quantitative composition:
- 20-87% by weight of Irbesartan;
- 10-25% by weight of maltose;
- 1-10% by weight of a binder;
- 1-9% by weight of a disintegrant;
- 0-15% by weight of an additional diluent;
- 0.1-3% by weight of a glidant;
- 0.5-3% by weight of a lubricant;
- 0-15% by weight of a diuretic;
said percentages being with respect to the total weight of the formulation.

Optionally, the granules of the formulation may comprise part of the maltose or of the additional diluent. Preferably the granules are formulated without maltose or additional diluent.

The additional diluent used in the formulation of the present invention is selected from one or more of the list consisting of microcrystalline cellulose, cellulose powder, calcium hydrogen phosphate dihydrate, starch, maltose and a functional equivalent, excluding lactose monohydrate and anhydrous lactose. Preferably, the diluent is microcrystalline cellulose.

The binder used in the formulation of the present invention is selected from one or more of the list consisting of povidone, hydroxypropylmethyl cellulose, pregelatinized starch, hydroxypropyl cellulose LH-21, starch and a functional equivalent.

The disintegrant used in the formulation of the present invention is selected from one or more of the list consisting of croscarmellose sodium, crospovidone, carboxymethyl cellulose sodium, carboxymethyl starch sodium and a functional equivalent.

The glidant used in the formulation of the present invention is selected from one or more of the list consisting of silica colloidal anhydrous, magnesium trisilicate, talc and a functional equivalent.

The lubricant used in the formulation of the present invention is selected from one or more of the list consisting of magnesium stearate, stearic acid, glycerol dibehenate, talc and a functional equivalent.

The Irbesartan used in the oral pharmaceutical formulations of the present invention is, preferably, Irbesartan polymorphic Form A as described in EP0708103.

In a particular embodiment of the invention, when a diuretic is incorporated in the pharmaceutical formulation, said diuretic can be inside and/or out of the Irbesartan granules. Preferably, said diuretic is hydrochlorothiazide (HCTZ).

In a preferred embodiment, the oral formulation of the invention is in the form of a tablet.

In another particular embodiment, the oral formulation of the invention is further coated.

In another aspect, the present invention relates to a process for preparing an oral pharmaceutical formulation as defined above using a wet granulation process, which comprises mixing and granulating the Irbesartan, the disintegrant and, optionally, part of the binder with water or a water solution of the binder.

Optionally the Irbesartan can be granulated with part of the maltose or of the additional diluent.

In a particular embodiment, 0-10% by weight of the binder is mixed with the Irbesartan and 90-100% by weight of the binder is used in the binder water solution. More preferably, all the binder is present in the binder water solution.

In another particular embodiment, the process for preparing the Irbesartan oral pharmaceutical formulations of the present invention further comprises:
a) mixing the granules obtained by the process as defined above, with the maltose, the glidant, the disintegrant and, optionally, the additional diluent and the optional diuretic;
b) mixing the mixture obtained in step a) with the lubricant;
c) compressing the resulting mixture obtained in step b) to prepare a tablet; and
d) optionally, coating the tablet.

Even in another particular embodiment, when a diuretic is incorporated into the granules of the pharmaceutical formulation, the process for the preparation of said formulation further comprises mixing and granulating with the diuretic.

In this case, the process for preparing the Irbesartan and diuretic oral pharmaceutical formulations of the present invention further comprises:
a) mixing the granules obtained by the process as defined above, with the maltose, the glidant, the disintegrant and, optionally, the additional diluent;
b) mixing the mixture obtained in step a) with the lubricant;
c) compressing the resulting mixture obtained in step b) to prepare a tablet; and
d) optionally, coating the tablet.

In the following, the present invention is further illustrated by examples. They should in no case be interpreted as a limitation of the scope of the invention as defined in the claims.

### EXAMPLES

### Example 1:

### Quantitative composition:

| | **(g)** | **%** |
|---|---|---|
| Irbesartan | 75,00 | 72,82 |
| Lactose monohydrate | 12,31 | 11,96 |
| Povidone | 5,20 | 5,00 |
| Croscarmellose sodium | 3,10 | 3,01 |
| Microcrystalline Cellulose | 6,10 | 5,93 |
| Anhydrous colloidal silica | 0,26 | 0,25 |
| Magnesium stearate | 1,03 | 1,00 |
| Purified water * | q.s. | |
| Total weight | 103 | |

| | | |
|---|---|---|
| * solvent which disappears during the wet granulation process. | | |

The Irbesartan, the lactose, the povidone and the croscarmellose sodium were granulated with water. Next, the granules were dried and sieved. Said granules were mixed with the cellulose microcrystalline, anhydrous colloidal silica and the magnesium stearate.

When trying to compress the mixture it resulted in low flowability and impossible to compress the mixture.

### Example 2:

Using the same quantitative composition as in example 1, the Irbesartan, the lactose, the povidone and half of the croscarmellose sodium were sieved, mixed and granulated with water. Next, the granulate was dried and sieved.

The remaining croscarmellose sodium, the microcrystalline cellulose and the anhydrous colloidal silica were sieved and mixed together and then were mixed with the previously prepared granules. Finally, the magnesium stearate was added and mixed. When trying to compress the mixture it resulted in low flowability and impossible to compress the mixture.

### Example 3:

Using the same quantitative composition as in example 1, the Irbesartan, the lactose and half of the croscarmellose sodium were sieved, mixed and granulated with a water solution of povidone. Next, the granulate was dried and sieved.

The remaining croscarmellose sodium, the microcrystalline cellulose and the anhydrous colloidal silica were sieved and mixed together and then were mixed with the previously formed granules. Finally, the magnesium stearate was added and mixed. When trying to compress the mixture it resulted in low flowability and impossible to compress the mixture.

### Example 4:

Using the same quantitative composition as in example 1, granules of Irbesartan where prepared with the Irbesartan, half the lactose and half of the croscarmellose sodium were sieved, mixed and granulated with a water solution of povidone. Next, the granulate was dried and sieved.

The remaining lactose and croscarmellose sodium, the microcrystalline cellulose and the anhydrous colloidal silica were sieved and mixed together and then were mixed with the previously formed granules. Finally, the magnesium stearate was added and mixed. When trying to compress the mixture it resulted in low flowability and impossible to obtain homogeneous tablets.

### Example 5:

### Quantitative composition:

| | **(mg)** | **%** |
|---|---|---|
| Granules | | |
| Irbesartan | 75,00 | 60,05 |
| Lactose | 25,36 | 20,31 |
| Povidone | 6,44 | 5,16 |
| Croscarmellose sodium | 3,86 | 3,09 |
| Microcrystalline Cellulose | 12,62 | 10,10 |
| Anhydrous colloidal silica | 0,32 | 0,26 |
| Magnesium stearate | 1,29 | 1,03 |
| Purified water* | q.s. | |
| Total weight | 125 | |

| | | |
|---|---|---|
| * solvent which disappears during the wet granulation process. | | |

The Irbesartan and half of the croscarmellose sodium were sieved, mixed and granulated with a water solution of povidone. Next, the granules were dried and sieved.

The remaining croscarmellose sodium, the lactose, the microcrystalline cellulose and the anhydrous colloidal silica were sieved and mixed together and then were mixed with the previously formed granules. Finally, the magnesium stearate was added and mixed. When trying to compress the mixture it resulted in low flowability and impossible to obtain homogeneous tablets.

### Example 6:

### Quantitative composition:

| | **(mg)** | **%** |
|---|---|---|
| Granules | | |
| Irbesartan | 75,00 | 60,00 |
| Maltose | 12,73 | 10,18 |
| Povidone | 6,44 | 5,15 |
| Croscarmellose sodium | 1,93 | 1,54 |
| Purified water* | q.s. | |
| Extragranular | | |
| Maltose | 12,73 | 10,18 |
| Croscanmellose sodium | 1,93 | 1,54 |
| Microcrystalline Cellulose | 12,62 | 10,10 |
| Anhydrous colloidal silica | 0,32 | 0,26 |
| Magnesium stearate | 1,30 | 1,04 |
| Total weight | 125 | |

| | | |
|---|---|---|
| * solvent which disappears during the wet granulation process. | | |

Following the procedure in Example 4 homogeneous tablets were prepared. The mixture showed good flowability properties, and the tablets could be properly compressed. The resulting tablets were homogeneous.

Open Dish studies were performed on the resulting tablets at 30 days, the tablets dissolved in less than 30 minutes, and maintained good crushing and friability properties.

### Example 7:

### Quantitative composition:

| | **(mg)** | **%** |
|---|---|---|
| Granules | | |
| Irbesartan | 75,00 | 58,25 |
| Povidone | 6,44 | 5,00 |
| Croscarmellose sodium | 1,93 | 1,50 |
| Purified water* | q.s. | |
| Extragranular | | |
| Maltose | 25,47 | 19,78 |
| Croscarmellose sodium | 1,93 | 1,50 |
| Microcrystalline Cellulose | 12,62 | 9,80 |
| Anhydrous colloidal silica | 0,32 | 0,25 |
| Magnesium stearate | 1,29 | 1,00 |
| Coating | 3,75 | 2,91 |
| Total weight | 128,75 | |

| | | |
|---|---|---|
| * solvent which disappears during the wet granulation process. | | |

Following the procedure in Example 5 homogeneous tablets were prepared. The mixture showed good flowability properties, and the tablets could be properly compressed. The resulting tablets were homogeneous.

Open Dish studies were performed on the resulting tablets at 30 days, the tablets dissolved in less than 30 minutes, and maintained good crushing and friability

### Example 8:

### Quantitative composition:

| | **(mg)** | **%** |
|---|---|---|
| Irbesartan | 75,00 | 53,19 |
| Maltose | 25,36 | 17,99 |
| Povidone | 6,45 | 4,57 |
| Croscarmellose sodium | 3,71 | 2,63 |
| Microcrystalline Cellulose | 12,62 | 8,95 |
| Anhydrous colloidal silica | 0,32 | 0,23 |
| Magnesium stearate | 1,29 | 0,91 |
| HCTZ | 12,50 | 8,87 |
| Coating | 3,75 | 2,66 |
| Purified water* | q.s. | |
| Total weight | 141 | |

| | | |
|---|---|---|
| * solvent which disappears during the wet granulation process. | | |

Following the procedure in Example 5, including the hydrochlorothiazide (HCTZ) out of the granules, homogeneous tablets were prepared. The mixture showed good flowability properties, and the tablets could be properly compressed. The resulting tablets were homogeneous.

Open Dish studies were performed on the resulting tablets at 30 days, the tablets dissolved in less than 30 minutes, and maintained good crushing and friability properties.

The following variations were performed to examples 6, 7 and 8.

Instead of microcrystalline cellulose other diluents were tested: cellulose powder, calcium hydrogen phosphate dihydrate, starch and maltose. Similar results were obtained.

Instead of povidone other binders were tested: hydroxypropylmethyl cellulose, pregelatinized starch, hydroxypropyl cellulose LH-21 and starch. Similar results were obtained.

Instead of croscarmellose sodium other disintegrants were tested: crospovidone, carboxymethyl cellulose sodium and carboxymethyl starch sodium. Similar results were obtained.

Instead of silica colloidal anhydrous other glidants were tested: magnesium trisilicate and talc. Similar results were obtained.

Instead of magnesium stearate other lubricants were tested: stearic acid, glyceryl behenate and talc. Similar results were obtained.

## Claims

1. A pharmaceutical composition comprising Irbesartan **characterized in that** it includes maltose.

2. An oral pharmaceutical formulation comprising a pharmaceutical composition as defined in claim 1.

3. The formulation according to claim 2 which comprises:
a. granules comprising Irbesartan, a binder and a disintegrant; and
b. a mixture of maltose, a disintegrant, a glidant, a lubricant and, optionally, an additional diluent.

4. The formulation according to claim 3 which further comprises a diuretic inside the granules and/or out of the granules.

5. The formulation according to claims 3 or 4, wherein the granules comprise:
- 67-97.5% by weight of Irbesartan;
- 2-12% by weight of a binder;
- 0.5-6% by weight of a disintegrant; and
- 0-15% of a diuretic;
with respect to the total weight of the granules.

6. The formulation according to any of claims 3 to 5, **characterized by** the following total quantitative composition:
- 20-87% by weight of Irbesartan;
- 10-25% by weight of maltose;
- 1-10% by weight of a binder;
- 1-9% by weight of a disintegrant;
- 0-15% by weight of an additional diluent;
- 0.1-3% by weight of a glidant;
- 0.5-3% by weight of a lubricant;
- 0-15% by weight of a diuretic.
with respect to the total weight of the formulation.

7. The formulation according to any of claims 3 to 6, wherein the additional diluent is selected from one or more of the list consisting of microcrystalline cellulose, cellulose powder, calcium hydrogen phosphate dihydrate, starch, maltose and a functional equivalent, excluding lactose monohydrate and anhydrous lactose; more particularly the diluent is microcrystalline cellulose.

8. The formulation according to any of claims 3 to 7, wherein the binder is selected from one or more of the list consisting of povidone, hydroxypropylmethyl cellulose, pregelatinized starch, hydroxypropyl cellulose LH-21, starch and a functional equivalent.

9. The formulation according to any of claims 3 to 8, wherein the disintegrant is selected from one or more of the list consisting of croscarmellose sodium, crospovidone, carboxymethyl cellulose sodium, carboxymethyl starch sodium and a functional equivalent.

10. The formulation according to any of claims 3 to 9, wherein the glidant is selected from one or more of the list consisting of silica colloidal anhydrous, magnesium trisilicate, talc and a functional equivalent.

11. The formulation according to any of claims 3 to 10, wherein the lubricant is selected from one or more of the list consisting of magnesium stearate, stearic acid, glycerol dibehenate, talc and a functional equivalent.

12. The formulation according to any of claims 4 to 11, wherein the diuretic is hydrochlorothiazide.

13. The formulation according to any of claims 2 to 12, wherein the Irbesartan is Irbesartan polymorphic Form A.

14. The formulation according to any of claims 3 to 13 wherein the granules comprise part of the maltose or part of the additional diluent.

15. The formulation according to any of claims 2 to 14, which is in the form of a tablet.

16. The formulation according to any of claims 2 to 15, wherein said formulation is further coated.

17. A process for preparing an oral pharmaceutical formulation as defined in any of claims 2 to 16, which comprises mixing and granulating the Irbesartan, the disintegrant and, optionally, part of the binder with water or a water solution of the binder.

18. The process according to claim 17, wherein 0-10% by weight of the binder is mixed with the Irbesartan and 90-100% by weight of the binder is used in the binder water solution.

19. The process according to claim 18 wherein all the binder is present in the binder water solution.

20. The process according to claims 17 to 19 wherein the Irbesartan is further granulated with part of the maltose or part of the additional diluent.

21. The process according to any of claims 17 to 20 which further comprises:
a. mixing the granules obtained as defined in any of claims 17 to 20 with the maltose, the glidant, the disintegrant and, optionally, with the additional diluent and the optional diuretic;
b. mixing the mixture obtained in step a) with the lubricant;
c. compressing the resulting mixture obtained in step b) to prepare a tablet; and
d. optionally, coating the tablet.

22. The process according to any of claims 17 to 20 which further comprises mixing and granulating with a diuretic.

23. The process according to claim 22 which further comprises:
a. mixing the granules obtained as defined in claim 22 with the maltose, the glidant, the disintegrant and, optionally, the additional diluent;
b. mixing the mixture obtained in step a) with the lubricant;
c. compressing the resulting mixture obtained in step b) to prepare a tablet; and
d. optionally, coating the tablet.

## Patentansprüche

1. Pharmazeutische Zusammensetzung, umfassend Irbesartan, **dadurch gekennzeichnet, dass** sie Maltose umfasst.

2. Orale pharmazeutische Formulierung, umfassend eine pharmazeutische Zusammensetzung gemäss Anspruch 1.

3. Formulierung gemäss Anspruch 2, umfassend:
(a) Körnchen, die Irbesartan, ein Bindemittel und ein Zerfallsmittel umfassen; und
(b) eine Mischung von Maltose, einem Zerfallsmittel, einem Fliessreguliermittel, einem Gleitmittel und optional einem zusätzlichen Verdünnungsmittel.

4. Formulierung gemäss Anspruch 3, die ferner ein Diuretikum innerhalb der Körnchen und/oder ausserhalb der Körnchen umfasst.

5. Formulierung gemäss Anspruch 3 oder 4, worin die Körnchen umfassen:
- 67 bis 97,5 Gew.% Irbesartan;
- 2 bis 12 Gew.% Bindemittel;
- 0,5 bis 6 Gew.% Zerfallsmittel; und
- 0 bis 15 % Diuretikum;
bezogen auf das Gesamtgewicht der Körnchen.

6. Formulierung gemäss irgendeinem der Ansprüche 3 bis 5, **gekennzeichnet durch** die folgende Gesamtmengenzusammensetzung:
- 20 bis 87 Gew.% Irbesartan;
- 10 bis 25 Gew.% Maltose;
- 1 bis 10 Gew.% Bindemittel;
- 1 bis 9 Gew.% Zerfallsmittel;
- 0 bis 15 Gew.% zusätzliches Verdünnungsmittel;
- 0,1 bis 3 Gew.% Fliessreguliermittel;
- 0,5 bis 3 Gew.% Gleitmittel;
- 0 bis 15 Gew.% Diuretikum;
bezogen auf das Gesamtgewicht der Formulierung.

7. Formulierung gemäss irgendeinem der Ansprüche 3 bis 6, worin das zusätzliche Verdünnungsmittel ausgewählt ist aus einem oder mehreren der Aufzählung, bestehend aus mikrokristalliner Cellulose, Cellulosepulver, Calczumhydrogenphosphatdihydrat, Stärke, Maltose und einem funktionellen Äquivalent, ausser Lactosemonohydrat und wasserfreier Lactose; worin insbesondere das Verdünnungsmittel mikrokristalline Cellulose ist.

8. Formulierung gemäss irgendeinem der Ansprüche 3 bis 7, worin das Bindemittel ausgewählt ist aus einem oder mehreren der Aufzählung, bestehend aus Povidon, Hydroxypropylmethylcellulose, vorgelatinisierter Stärke, Hydroxypropylcellulose, LH-21, Stärke und einem funktionellen Äquivalent.

9. Formulierung gemäss irgendeinem der Ansprüche 3 bis 8, worin das Zerfallsmittel ausgewählt ist aus einem oder mehreren der Aufzählung, bestehend aus Croscarmellosenatrium, Crospovidon, Carboxymethylcellulosenatrium, Carboxymethylstärkenatrium und einem funktionellen Äquivalent.

10. Formulierung gemäss irgendeinem der Ansprüche 3 bis 9, worin das Fliessreguliermittel ausgewählt ist aus einem oder mehreren der Aufzählung, bestehend aus wasserfreiem kolloidalen Silica, Magnesiumtrisilicat, Talk und einem funktionellen Äquivalent.

11. Formulierung gemäss irgendeinem der Ansprüche 3 bis 10, worin das Gleitmittel ausgewählt ist aus einem oder mehreren der Aufzählung, bestehend aus Magnesiumstearat, Stearinsäure, Glycerindibehenat, Talk und einem funktionellen Äquivalent.

12. Formulierung gemäss irgendeinem der Ansprüche 4 bis 11, worin das Diuretikum Hydrochlorthiazid ist.

13. Formulierung gemäss irgendeinem der Ansprüche 2 bis 12, worin das Irbesartan Irbesartan der polymorphen Form A ist.

14. Formulierung gemäss irgendeinem der Ansprüche 3 bis 13, worin die Körnchen einen Teil der Maltose oder einen Teil des zusätzlichen Verdünnungsmittels umfassen.

15. Formulierung gemäss irgendeinem der Ansprüche 2 bis 14, die in Form einer Tablette ist.

16. Formulierung gemäss irgendeinem der Ansprüche 2 bis 15, worin die Formulierung ferner beschichtet ist.

17. Verfahren zur Herstellung einer oralen pharmazeutischen Formulierung gemäss irgendeinem der Ansprüche 2 bis 16, das das Mischen und Granulieren von Irbesartan, dem Zerfallmittel und optional einem Teil des Bindemittels mit Wasser oder einer Wasserlösung des Bindemittels umfasst.

18. Verfahren gemäss Anspruch 17, worin 0 bis 10 Gew.% des Bindemittels mit Irbesartan vermischt werden und 90 bis 100 Gew.% des Bindemittels in der Bindemittel-Wasserlösung verwendet werden.

19. Verfahren gemäss Anspruch 18, worin das gesamte Bindemittel in der Bindemittel-Wasserlösung vorliegt.

20. Verfahren gemäss den Ansprüchen 17 bis 19, worin das Irbesartan ferner mit einem Teil der Maltose oder einem Teil des zusätzlichen Verdünnungsmittels granuliert wird.

21. Verfahren gemäss irgendeinem der Ansprüche 17 bis 20, das ferner umfasst:
(a) Mischen der wie in irgendeinem der Ansprüche 17 bis 20 definiert erhaltenen Körnchen mit der Maltose, dem Fliessreguliermittel, dem Zerfallsmittel und optional dem zusätzlichen Verdünnungsmittel und dem optionalen Diuretikum;
(b) Mischen der in Schritt (a) erhaltenen Mischung mit dem Gleitmittel;
(c) Komprimieren der in Schritt (b) erhaltenen resultierenden Mischung, um eine Tablette herzustellen; und
(d) optional Beschichten der Tablette.

22. Verfahren gemäss irgendeinem der Ansprüche 17 bis 20, das ferner das Mischen und Granulieren mit einem Diuretikum umfasst.

23. Verfahren gemäss Anspruch 22, das ferner umfasst:
(a) Mischen der wie in Anspruche 22 definiert erhaltenen Körnchen mit der Maltose, dem Fliessreguliermittel, dem Zerfallsmittel und optional dem zusätzlichen Verdünnungsmittel;
(b) Mischen der in Schritt (a) erhaltenen Mischung mit dem Gleitmittel;
(c) Komprimieren der in Schritt (b) erhaltenen resultierenden Mischung, um eine Tablette herzustellen; und
(d) optional Beschichten der Tablette.

## Revendications

1. Composition pharmaceutique comprenant de l'irbésartan, **caractérisée en ce qu'**elle comprend du maltose.

2. Formulation pharmaceutique orale comprenant une composition pharmaceutique telle qu'elle est définie dans la revendication 1.

3. Formulation selon la revendication 2, qui comprend :
a. des granulés comprenant de l'irbésartan, un liant et un délitant ; et
b. un mélange de maltose, d'un délitant, d'un agent de glissement, d'un lubrifiant et, facultativement, d'un diluant supplémentaire.

4. Formulation selon la revendication 3, qui comprend en outre un diurétique à l'intérieur des granulés et/ou à l'extérieur des granulés.

5. Formulation selon les revendications 3 ou 4, dans laquelle les granulés comprennent :
- 67 à 97,5 % en poids d'irbésartan ;
- 2 à 12 % en poids d'un liant ;
- 0,5 à 6 % en poids d'un délitant ; et
- 0 à 15 % en poids d'un diurétique ;
par rapport au poids total des granulés.

6. Formulation selon l'une quelconque des revendications 3 à 5, **caractérisée par** la composition quantitative totale suivante :
- 20 à 87 % en poids d'irbésartan ;
- 10 à 25 % en poids de maltose ;
- 1 à 10 % en poids d'un liant ;
- 1 à 9 % en poids d'un délitant ;
- 0 à 15 % en poids d'un diluant supplémentaire ;
- 0,1 à 3 % en poids d'un agent de glissement ;
- 0,5 à 3 % en poids d'un lubrifiant ;
- 0 à 15 % en poids d'un diurétique ;
par rapport au poids total de la formulation.

7. Formulation selon l'une quelconque des revendications 3 à 6, dans laquelle le diluant supplémentaire est sélectionné parmi un ou plusieurs diluants de la liste constituée par la cellulose microcristalline, la poudre de cellulose, l'hydrogénophosphate de calcium dihydraté, l'amidon, le maltose et un équivalent fonctionnel, sauf le lactose monohydraté et le lactose anhydre ; plus particulièrement le diluant est la cellulose microcristalline.

8. Formulation selon l'une quelconque des revendications 3 à 7, dans laquelle le liant est sélectionné parmi un ou plusieurs liants de la liste constituée par la povidone, l'hydroxypropylméthylcellulose, l'amidon prégélatinisé, l'hydroxypropylcellulose LH-21, l'amidon et un équivalent fonctionnel.

9. Formulation selon l'une quelconque des revendications 3 à 8, dans laquelle le délitant est sélectionné parmi un ou plusieurs délitants de la liste constituée par la croscarmellose de sodium, la crospovidone, la carboxyméthylcellulose de sodium, le carboxyméthylamidon de sodium et un équivalent fonctionnel.

10. Formulation selon l'une quelconque des revendications 3 à 9, dans laquelle l'agent de glissement est sélectionné parmi un ou plusieurs agents de glissement de la liste constituée par la silice colloïdale anhydre, le trisilicate de magnésium, le talc et un équivalent fonctionnel.

11. Formulation selon l'une quelconque des revendications 3 à 10, dans laquelle le lubrifiant est sélectionné parmi un ou plusieurs lubrifiants de la liste constituée par le stéarate de magnésium, l'acide stéarique, le dibéhénate de glycérol, le talc et un équivalent fonctionnel.

12. Formulation selon l'une quelconque des revendications 4 à 11, dans laquelle le diurétique est l'hydrochlorothiazide.

13. Formulation selon l'une quelconque des revendications 2 à 12, dans laquelle l'irbésartan est la forme polymorphe A de l'irbésartan.

14. Formulation selon l'une quelconque des revendications 3 à 13, dans laquelle les granulés comprennent une partie du maltose ou une partie du diluant supplémentaire.

15. Formulation selon l'une quelconque des revendications 2 à 14, qui se présente sous la forme d'un comprimé.

16. Formulation selon l'une quelconque des revendications 2 à 15, dans laquelle ladite formulation est en outre enrobée.

17. Procédé de préparation d'une formulation pharmaceutique orale selon l'une quelconque des revendications 2 à 16, qui comprend le mélange et la granulation de l'irbésartan, du délitant et, facultativement, d'une partie du liant avec de l'eau ou une solution aqueuse du liant.

18. Procédé selon la revendication 17, dans lequel 0 à 10 % en poids du liant sont mélangés avec l'irbésartan et 90 à 100 % en poids du liant sont utilisés dans la solution aqueuse du liant.

19. Procédé selon la revendication 18, dans lequel la totalité du liant est présente dans la solution aqueuse du liant.

20. Procédé selon les revendications 17 à 19, dans lequel l'irbésartan est en outre granulé avec une partie du maltose ou une partie du diluant supplémentaire.

21. Procédé selon l'une quelconque des revendications 17 à 20, qui comprend en outre :
a. le mélange des granulés obtenus selon l'une quelconque des revendications 17 à 20 avec le maltose, l'agent de glissement, le délitant et, facultativement, avec le diluant supplémentaire et le diurétique facultatif ;
b. le mélange du mélange obtenu à l'étape a. avec le lubrifiant ;
c. la compression du mélange résultant obtenu à l'étape b. pour préparer un comprimé ; et
d. facultativement, l'enrobage du comprimé.

22. Procédé selon l'une quelconque des revendications 17 à 20, qui comprend en outre le mélange et la granulation avec un diurétique.

23. Procédé selon la revendication 22, qui comprend en outre :
a. le mélange des granulés obtenus selon la revendication 22 avec le maltose, l'agent de glissement, le délitant et, facultativement, le diluant supplémentaire ;
b. le mélange du mélange obtenu à l'étape a. avec le lubrifiant ;
c. la compression du mélange résultant obtenu à l'étape b. pour préparer un comprimé ; et
d. facultativement, l'enrobage du comprimé.
